# EUROPEAN PATENT APPLICATION

(11) **EP 4 678 176 A1**
(43) Date of publication of application: **14.01.2026**
(21) Application number: 24382746.6
(22) Date of filing: 11.07.2024
(51) Int. Cl.: A61K 31/665, A61K 31/198, A61P 31/04, C07F 9/38, A61K 31/4172

(54) **AMINO ACID SALTS OF FOSFOMYCIN AND COMPOSITIONS THEREOF**

(71) Applicant: Ercros, S.A., 08024 Barcelona (ES); LABORATORIOS ERN S.A., 08020 Barcelona (ES)
(72) Inventor: SOLANES VALERO, Irene, 08020 BARCELONA (ES); MARIÑO BERMÚDEZ, Marta, 08013 BARCELONA (ES); DE LA CRUZ ESCUDERO, Angel, 28300 ARANJUEZ (ES); DÍEZ ESCANCIANO, Luisa, 28300 ARANJUEZ (ES); MAS TORRENT, Gemma, 08014 BARCELONA (ES); LLOVERAS MONSERRAT, Maia, 08172 SANT CUGAT DEL VALLÈS (ES)
(74) Representative: ZBM Patents - Zea, Barlocci & Markvardsen

(57) **Abstract**

It refers to pharmaceutical or veterinary composition which comprises a therapeutically effective amount of a crystalline form of an amino acid salt of fosfomycin, together with one or more pharmaceutically or veterinary acceptable excipients, wherein the amino acid is lysine or histidine. It also refers to processes for the preparation of such crystalline forms and to the use of the same in injectable preparations for parenteral administration.

## Description

### Technical Field

The present invention relates to crystalline amino acid salts of fosfomycin and to pharmaceutical compositions comprising crystalline aminoacid salts of fosfomycin as an active pharmaceutical ingredient (API). Furthermore, the present invention relates to methods of producing such salts, as well as to their use in therapy, in particular for use in parenteral administration.

### Background Art

Fosfomycin is an antibiotic agent discovered in 1969 by CEPA (Compañía Española de Penicilina y Antibióticos) and Merck Sharp & Dome.

Fosfomycin is a broad-spectrum bactericidal antibiotic active against a wide range of gram-positive and gram-negative organisms. It inhibits bacterial cell wall biogenesis by interfering with peptidoglycan biosynthesis. It is a natural product produced by certain Streptomyces species, although nowadays it is produced by chemical synthesis.

Fosfomycin is clinically indicated in the treatment of urinary tract infections, mainly caused by Escherichia coli and Enterococcus faecalis, where it is administered at high doses. Combinations of fosfomycin with aminoglycosides or other drugs such as N-acetylcysteine have been explored for treatment of bacterial respiratory infections as well as ophthalmic and dermatological infections.

Its molecular structure corresponds to a phosphonic acid containing an oxirane 3-member ring [(-)(1R, 2S)-1,2-epoxypropylphosphonic acid] (see formula (I)).

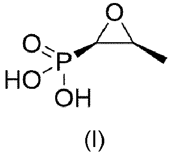

From a chemical point of view, an oxirane ring is rather labile under acidic and basic conditions. Fosfomycin is commonly formulated as a salt, such as disodium, calcium or tromethamine (trometamol; 1,3-dihydroxy-2-(hydroxymethyl)propan-2-yl) phosphonate salts. Calcium and tromethamine salts are mostly for oral use, whereas the disodium salt is indicated for intravenous administration.

The drug is generally well tolerated and has a low incidence of harmful side effects. However, disodium fosfomycin salt contains a high sodium load (25.3% in weight; 1 g of disodium fosfomycin contains 253 mg of sodium ion) which may cause electrolyte imbalances such as hypernatremia or hypokalemia. This could raise a problem for patients with cardiovascular, renal or hypertension diseases. Therefore, as a precaution, it is neither used in elderly patients. The clinical use of fosfomycin against multi-drug resistant bacteria is strongly limited due to high sodium uptake. Moreover, the low solubility of calcium fosfomycin salt results in low bioavailability.

ES359542 describes the resolution of racemic fosfomycin acid or a salt thereof through the precipitation of a diastereoisomeric salt with an optically active salt-forming compound. In the examples, they use (+)-Lysine to separate (-)-(cis-1,2-epoxypropyl)phosphonic acid (fosfomycin) as a salt. In the hand of the inventors the disclosed salts could not be obtained in a reproducible manner. Besides, this document does not disclose pharmaceutical formulations.

ES477167 relates to amino acid salts of fosfomycin, including lysine ones, and their preparation from fosfomycin in acid form. Lysine salt of fosfomycin is disclosed as an amorphous product.

ES483027 also relates to amino acid salts of fosfomycin prepared by epoxidation of the corresponding salts of cis-propenyl phosphonic acid. Lysine salt of fosfomycin is included within the examples although in the hand of the inventors it could not be obtained in a reproducible manner. Besides, this document does not disclose pharmaceutical formulations.

Therefore, there is still a need to provide alternative fosfomycin compositions that avoid the drawbacks and improve the treatments against bacterial infections.

### Summary of Invention

Despite the teachings of the above prior art documents, the present inventors have been able to provide crystalline amino acid salts of fosfomycin selected from lysine and histidine, particularly in their L form as shown in the general formula (II), which can be formulated into pharmaceutical and/or veterinary compositions which can be used as an antibiotic for parenteral administration.

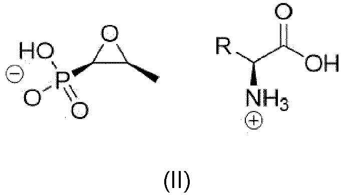

The formulations containing these crystalline amino acid salts of fosfomycin represent an advantage with respect to the current method of treatment, which comprises the use of disodium salt of fosfomycin. The latter is contraindicated in patients with cardiovascular and/or renal pathologies, as well as elderly people, since it increases sodium concentration in blood.

The crystalline lysine and histidine salts of the present invention show physicochemical properties (solubility, ions uptake, pH) which are more adequate for parenteral administration, in particular for the above-mentioned group of patients (i.e., elderly, with cardiovascular and/or renal pathologies, etc.).

The solid form of a drug substance or active pharmaceutical ingredient (API) used in a pharmaceutical formulation or medicament is very important for solubility reasons mentioned above, but also regarding dissolution rate, bioavailability, hygroscopicity and/or stability differences that may exist between the different solid forms. Thus, the existence of various solid forms, such as polymorphism or pseudo polymorphism can affect the properties of the drug product, meaning that a specific crystal form might be preferred over another one. Furthermore, certain crystalline forms may be preferred depending on the specific formulation or application.

Accordingly, a first aspect of the present invention is a pharmaceutical or veterinary composition which comprises a therapeutically effective amount of a crystalline form of an amino acid salt of fosfomycin, together with one or more pharmaceutically or veterinary acceptable excipients, wherein the amino acid is selected from the group consisting of lysine and histidine.

A second aspect of the present invention is a crystalline form of an amino acid salt of fosfomycin, wherein the amino acid is selected from the group consisting of lysine and histidine.

A third aspect of the present invention relates to a histidine salt of fosfomycin.

Furthermore, the inventors have surprisingly found that, after parenteral administration in rats and compared with disodium salt of fosfomycin, the concentration of the histidine salt of fosfomycin in urine is higher. The same applies to the lysine salt. Thus, the use of lysine and/or histidine salts of fosfomycin in an injectable preparation is highly convenient.

A fourth aspect of the present invention relates to the pharmaceutical or veterinary composition as defined herein, wherein the amino acid is selected from the group consisting of lysine and histidine, the crystalline forms as defined herein, or the histidine salt of fosfomycin, for parenteral use in therapy.

A fifth aspect of the present invention relates to the pharmaceutical or veterinary composition as defined herein, wherein the amino acid is selected from the group consisting of lysine and histidine, the crystalline forms as defined herein, or the histidine salt of fosfomycin, for use as antibiotic.

A sixth aspect of the present invention is to provide a process for the preparation of the crystalline form of an amino acid salt of fosfomycin as defined herein, wherein the amino acid is lysine, and which comprises:
i) providing a lysine salt of fosfomycin, and
ii) recrystallizing it in a mixture of water and ethanol in a volume ratio from 1:2 to 1:10, particularly about 1:5.

A seventh aspect of the present invention is to provide a process for the preparation of the crystalline form of an amino acid salt of fosfomycin as defined herein, wherein the amino acid is histidine, which comprises:
i) providing a histidine salt of fosfomycin, and
ii) recrystallizing it in a mixture of water and ethanol in a volume ratio from 1:2 to 1:15, particularly about 1:10.

An eighth aspect of the present invention is to provide a process for the preparation of the histidine salt of fosfomycin, which comprises:
a) providing a fosfomycin 1-amino-1-phenylethyl (PEA) salt,
b) treating the fosfomycin PEA salt with a base in aqueous media,
c) washing out the resulting aqueous solution with an organic solvent, and
d) adding histidine in a protonated form to the aqueous phase;
   or, alternatively,

a) providing a fosfomycin 1-amino-1-phenylethyl (PEA) salt, and
b) contacting the PEA salt with histidine as a free base in the presence of an acid in an aqueous alcoholic media;
   or, alternatively,

a) providing a fosfomycin 1-amino-1-phenylethyl (PEA) salt, and
b) contacting the PEA salt with histidine in protonated form in an aqueous alcoholic media; or, alternatively,

a) providing a fosfomycin 1-amino-1-phenylethyl (PEA) salt, and
b) contacting the PEA salt with an ionic exchange resin, and
c) reacting the resulting solution of step b) with histidine in an aqueous alcoholic media.

### Brief Description of Drawings

FIG. 1 shows XR-Diffractogram of crystalline lysine salt of fosfomycin.
FIG. 2 shows XR-Diffractogram of crystalline histidine salt of fosfomycin.
FIG.3 shows XR-Diffractogram of crystalline arginine salt of fosfomycin.
FIG. 4 shows electronic microscope image 600X of crystalline lysine salt of fosfomycin.
FIG. 5 shows electronic microscope image 100 X of crystalline histidine salt of fosfomycin.
FIG. 6 shows electronic microscope image 400 X of crystalline arginine salt of fosfomycin.

### Detailed description of the invention

All terms as used herein in this application, unless otherwise stated, shall be understood in their ordinary meaning as known in the art. Other more specific definitions for certain terms as used in the present application are as set forth below and are intended to apply throughout the description and claims.

As used herein, the indefinite articles "a" and "an" are synonymous with "at least one" or "one or more." Unless indicated otherwise, definite articles used herein, such as "the," also include the plural of the noun.

The term "about" or "around" or "approximately" as used herein refers to a range of values ± 10% of a specified value. For example, the expression "about 10" or "around 10" includes ± 10% of 10, i.e., from 9 to 11.

The word "comprise" for the purposes of the present invention encompasses the case of "consisting essentially of" and "consisting of".

The expression "therapeutically effective amount" as used herein, refers to the amount of a compound or in this case of an amino acid salt of fosfomycin that, when administered, is sufficient to prevent development of, or alleviate to some extent, one or more of the symptoms of the disease which is addressed. The particular dose of the amino acid salt of fosfomycin administered according to this invention will of course be determined by the particular circumstances surrounding the case, including salt administered, the route of administration, the particular condition being treated, and the similar considerations.

The expression "pharmaceutically acceptable excipients or carriers" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "patient" refers to a person who is designated to receive a medical treatment, therapy, or service. The term patient may be extended to an animal when used within the context of the animal receiving veterinary treatment or services.

The term "filtration" refers to the act of removing solid particles from a solution comprising a mixture of solid particles and liquid. It is understood that solid particles which are smaller than the particle size of the equipment used to filtrate the solution may not be separated from the mixture.

The term "crystallization" refers to any method known to a person skilled in the art to obtain from a solution a crystalline form of a solid product. An example of such methods may be the dissolution of the product in a single solvent or a combination of solvents, optionally at high temperature, followed by the removal of the solvent, the addition of an antisolvent or cooling of the dissolution to make the product precipitate. The conditions at which the crystallization is performed may influence of the form of the product obtained.

The term "room temperature" in the context of the present invention refers to a temperature from 15 °C to 30 °C, preferably from 20 °C to 25 °C.

The expression "obtainable by" is used herein for defining a product (i.e., the amino acid salt of fosfomycin) by its preparation process and refers to the product that can be obtained through the preparation process disclosed herein. For the purposes of the invention, the expressions "obtainable", "obtained" and similar equivalent expressions are used interchangeably and, in any case, the expression "obtainable" encompasses the expression "obtained".

As mentioned above, the present invention relates to a pharmaceutical or veterinary composition which comprises a therapeutically effective amount of a crystalline form of an amino acid salt of fosfomycin, together with one or more pharmaceutically or veterinary acceptable excipients, wherein the amino acid is selected from the group consisting of lysine and histidine.

In an embodiment, optionally in combination with any of the embodiments above or below, in the pharmaceutical or veterinary composition comprising a crystalline form of the lysine or histidine salt of fosfomycin according to the invention the crystalline form of an amino acid salt of fosfomycin comprises from 0.9 to 1.1 equivalents of amino acid relative to one equivalent of fosfomycin.

In a particular embodiment, optionally in combination with any of the embodiments above or below, the pharmaceutical or veterinary composition comprising a crystalline form of the lysine or histidine salt of fosfomycin according to the invention is an injectable preparation comprising a solution of the crystalline form of an amino acid salt of fosfomycin.

In a particular embodiment of the injectable preparation according to the invention, in combination with any of the embodiments above or below, the amino acid salt of fosfomycin is lysine salt of fosfomycin.

In a particular embodiment of the injectable preparation according to the invention, in combination with any of the embodiments above or below, the amino acid salt of fosfomycin is histidine salt of fosfomycin.

In a particular embodiment of the invention, in combination with any of the embodiments above or below, the amino acid salt of fosfomycin as described above is obtained as a sterile product. In a more particular embodiment, sterilization is achieved by a filtration, exposure to radiation or lyophilisation step.

It is also part of the invention a crystalline form of an amino acid salt of fosfomycin, wherein the amino acid is selected from the group consisting of lysine and histidine.

The amino acid salts of fosfomycin according to the invention have three chiral centres. Considering fosfomycin as a pure enantiomer, depending on the chiral center of the amino acid, meaning L and D (or R and S) enantiomers, two different diastereoisomeric salts may be obtained. Diastereoisomeric mixtures obtained can be separated into the pure isomers in a known manner on the basis of the physicochemical differences of the components, for example by fractional crystallization. Then, the desired isomer can be advantageously isolated.

Accordingly, in an embodiment of the crystalline form of amino acid salt of fosfomycin according to the invention, in combination with any of the embodiments above or below, the amino acid is selected from the group consisting of L-lysine and L-histidine. Formulas (III) and (IV) below represent L-lysine salt of Fosfomycin and L-histidine salt of fosfomycin respectively.

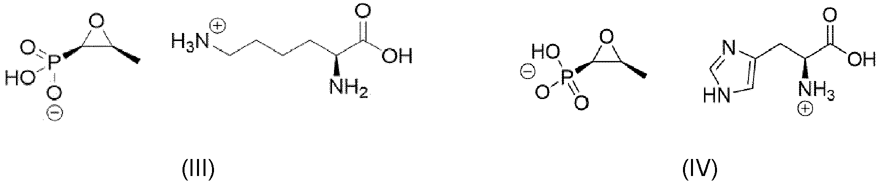

In a particular embodiment, optionally in combination with any of the embodiments above or below, the crystalline form of lysine salt of fosfomycin is L-lysine.

In another particular embodiment, optionally in combination with any of the embodiments above or below, the crystalline form of histidine salt of fosfomycin is L- histidine.

In an embodiment, optionally in combination with any of the embodiments above or below, the crystalline form of the lysine or histidine salt of fosfomycin according to the invention comprises from 0.9 to 1.1 equivalents of amino acid relative to one equivalent of fosfomycin, more particularly, the ratio of amino acid relative to fosfomycin is about 1:1.

In another embodiment, optionally in combination with any of the embodiments above or below, the crystalline form of the lysine salt of fosfomycin is characterized by an X-ray diffractogram that comprises characteristic peaks at 9.3, 19.6, 21.2, 23.7, and 24.1 ± 0.1 degrees 2 theta measured in an X-ray diffractometer with Cu Kα radiation (1.5406 Å).

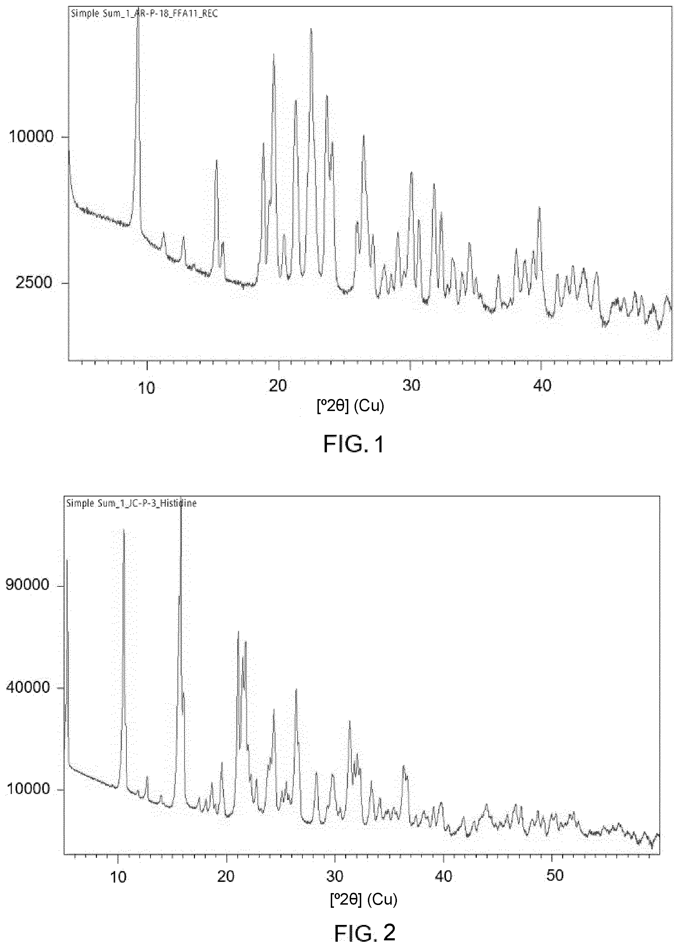

In another embodiment, optionally in combination with any of the embodiments above or below, the crystalline form of the lysine salt of fosfomycin is characterized by an X-ray diffractogram as shown in FIG. 1.

In another embodiment, optionally in combination with any of the embodiments above or below, the crystalline form of the lysine salt of fosfomycin is characterized by an X-ray diffractogram that comprises the characteristic peaks at the values shown in Table 1 ± 0.1 degrees 2 theta measured in an X-ray diffractometer with Cu Kα radiation (1.5406 Å).

**Table 1**

| Angle (2θ) | D Value (Å) | Rel. Intensity (%) | | Angle (2θ) | D Value (Å) | Rel. Intensity (%) |
|---|---|---|---|---|---|---|
| 9.2547 | 9.54820 | 92.70 | | 23.9717 | 3.70924 | 29.73 |
| 15.2891 | 5.79055 | 22.94 | | 24.1039 | 3.68920 | 40.54 |
| 18.8111 | 4.71357 | 37.92 | | 26.4124 | 3.37176 | 37.64 |
| 19.6200 | 4.52103 | 80.29 | | 30.1291 | 2.96375 | 27.23 |
| 21.2365 | 4.18039 | 51.06 | | 31.8083 | 2.81102 | 37.43 |
| 22.2263 | 3.99643 | 27.18 | | 31.8083 | 2.26140 | 28.50 |
| 23.6613 | 3.75719 | 65.33 | | | | |

In another embodiment, optionally in combination with any of the embodiments above or below, the crystalline form of the histidine salt of fosfomycin is characterized by an X-ray diffractogram that comprises characteristic peaks at 10.5, 15.8, 21.0, 21.7 and 26.4 ± 0.1 degrees 2 theta measured in an X-ray diffractometer with Cu Kα radiation (1.5406 Å).

In another embodiment, optionally in combination with any of the embodiments above or below, the crystalline form of the histdine salt of fosfomycin is characterized by an X-ray diffractogram as shown in FIG. 2.

In another embodiment, optionally in combination with any of the embodiments above or below, the crystalline form of the histidine salt of fosfomycin is characterized by an X-ray diffractogram that comprises the characteristic peaks at the values shown in Table 2 ± 0.1 degrees 2 theta measured in an X-ray diffractometer with Cu Kα radiation (1.5406 Å).

**Table 2**

| Angle (2θ) | D Value (Å) | Rel. Intensity (%) | | Angle (2θ) | D Value (Å) | Rel. Intensity (%) |
|---|---|---|---|---|---|---|
| 10.5012 | 8.41744 | 84.01 | | 21.7193 | 4.08854 | 35.57 |
| 15.6145 | 5.67061 | 46.11 | | 24.3534 | 3.65197 | 15.24 |
| 15.7671 | 5.61607 | 100.00 | | 26.4053 | 3.37265 | 21.40 |
| 16.0106 | 5.53117 | 16.69 | | 31.3502 | 2.85103 | 11.72 |
| 21.0393 | 4.21913 | 40.09 | | | | |
| 21.3863 | 4.15146 | 17.16 | | | | |
| 21.4970 | 4.13033 | 25.92 | | | | |

As mentioned above, the inventors have surprisingly found that histidine salt of fosfomycin shows greater solubility, and its concentration in urine is higher compared to fosfomycin sodium salt, among other advantages that make its use in an injectable preparation highly convenient.

Accordingly, the invention relates to a histidine salt of fosfomycin.

It is also part of the invention a pharmaceutical or veterinary composition comprising a therapeutically effective amount of histidine salt of fosfomycin in, together with one or more pharmaceutically or veterinary acceptable excipients.

In a particular embodiment, optionally in combination with any of the embodiments above or below, the pharmaceutical or veterinary composition comprising a histidine salt of fosfomycin according to the invention is an injectable preparation comprising a solution of the histidine salt of fosfomycin.

In an embodiment, optionally in combination with any of the embodiments above or below, the pharmaceutical or veterinary composition comprising histidine salt of fosfomycin as defined herein comprises from 0.9 to 1.1 equivalents of amino acid relative to one equivalent of fosfomycin, more particularly, the ratio of amino acid relative to fosfomycin is about 1:1.

It is also part of the invention a pharmaceutical or veterinary composition as defined herein, the crystalline forms as defined herein, or a histidine salt of fosfomycin, for parenteral use in therapy.

In an embodiment, optionally in combination with any of the embodiments above or below, the pharmaceutical or veterinary composition as defined herein, the crystalline forms as defined herein, or the histidine salt of fosfomycin, is for use as antibiotic in the treatment of bacterial infections.

This aspect may also be formulated as the use of a pharmaceutical or veterinary composition as defined herein, the crystalline forms as defined herein, and a histidine salt of fosfomycin, for the manufacture of a medicament for the treatment of bacterial infections.

The present invention also relates to a method for the treatment of bacterial infections, comprising administering a pharmaceutical or veterinary composition which comprises a therapeutically effective amount of either a crystalline form of an amino acid salt of fosfomycin as defined herein, or a histidine salt of fosfomycin, together with one or more pharmaceutically or veterinary acceptable excipients to a subject in need thereof, in particular a mammal such as a human being.

The invention also provides methods to obtain said crystalline forms of amino acid salts of fosfomycin. Thus, in another aspect of the invention is a process for the preparation of the crystalline form of an amino acid salt of fosfomycin as defined herein, wherein the amino acid is lysine, which comprises:
i) providing a lysine salt of fosfomycin, and
ii) recrystallizing it in a mixture of water and ethanol in a volume ratio is from 1:2 to 1:10, particularly about 1:5. In a particular embodiment the water and ethanol volume ratio is about 1:2, about 1:3, about 1:4, about 1:5, about 1:6, about 1:7, about 1:8, about 1:9, or about 1:10.

The invention also provides methods to obtain said crystalline forms of amino acid salts of fosfomycin. Thus, in another aspect of the invention is a process for the preparation of the crystalline form of an amino acid salt of fosfomycin as defined herein, wherein the amino acid is histidine, which comprises:
i) providing a histidine salt of fosfomycin, and
ii) recrystallizing it in a mixture of water and ethanol in a volume ratio is from 1:2 to 1:15, particularly about 1:10. In a particular embodiment the water and ethanol volume ratio is about 1:2, about 1:3, about 1:4, about 1:5, about 1:6, about 1:7, about 1:8, about 1:9, about 1:10, about 1:11, about 1:12, about 1:13, about 1:14 or about 1:15.

In an embodiment, optionally in combination with any of the embodiments above or below, the amino acid salt of fosfomycin of step (i) as defined above is prepared either by a process which comprises:
a) providing a fosfomycin 1-amino-1-phenylethyl (PEA) salt,
b) treating the fosfomycin PEA salt with a base in aqueous media,
c) washing out the resulting aqueous solution with an organic solvent, and
d) adding the corresponding amino acid in a protonated form to the aqueous phase;
or, alternatively, a process which comprises:
a) providing a fosfomycin 1-amino-1-phenylethyl (PEA) salt, and
b) contacting the PEA salt with the corresponding amino acid as a free base in the presence of an acid in an aqueous alcoholic media;
or, alternatively, a process which comprises:
a) providing a fosfomycin 1-amino-1-phenylethyl (PEA) salt, and
b) contacting the PEA salt with the corresponding amino acid in protonated form in an aqueous alcoholic media;
or, alternatively, a process which comprises:
a) providing a fosfomycin 1-amino-1-phenylethyl (PEA) salt, and
b) contacting the PEA salt with an ionic exchange resin, and
c) reacting the resulting solution of step b) with the corresponding amino acid in an aqueous alcoholic media.

Another aspect of the invention is a process for the preparation of the histidine salt of fosfomycin as defined herein, which comprises:
a) providing a fosfomycin 1-amino-1-phenylethyl (PEA) salt,
b) treating the fosfomycin PEA salt with a base in aqueous media,
c) washing out the resulting aqueous solution with an organic solvent, and
d) adding histidine in a protonated form to the aqueous phase;
or, alternatively, a process which comprises:
a) providing a fosfomycin 1-amino-1-phenylethyl (PEA) salt, and
b) contacting the PEA salt with histidine as a free base in the presence of an acid in an aqueous alcoholic media;
or, alternatively, a process which comprises:
a) providing a fosfomycin 1-amino-1-phenylethyl (PEA) salt, and
b) contacting the PEA salt with histidine in protonated form in an aqueous alcoholic media; or, alternatively,
a process which comprises:
a) providing a fosfomycin 1-amino-1-phenylethyl (PEA) salt, and
b) contacting the PEA salt with an ionic exchange resin, and
c) reacting the resulting solution of step b) with histidine in an aqueous alcoholic media.

In an embodiment of the above processes according to the invention, in combination with any of the embodiments above or below, the base used for treating the fosfomycin PEA salt is a hydroxide, such as sodium hydroxide.

In a particular embodiment, optionally in combination with any of the embodiments above or below, the amino acid in a protonated form is in hydro halogenated form, such as in hydrochloride form. In another particular embodiment, optionally in combination with any of the embodiments above or below, the organic solvent used for washing is a C₁-C₈ hydrocarbon, such as toluene, hexane, or heptane. In a more particular embodiment, optionally in combination with any of the embodiments above or below, the organic solvent is toluene.

In an embodiment of the above process according to the invention, in combination with any of the embodiments above or below, the acid used for treating the fosfomycin PEA salt is hydrochloric acid or a sulphonic acid. In a particular embodiment, optionally in combination with any of the embodiments above or below, the acid is p-toluenesulphonic acid.

In another embodiment of the processes according to the invention, in combination with any of the embodiments above or below, the aqueous alcoholic media is a mixture of water and one or more C₁-C₄-alcohols, such as methanol and ethanol, the ratio between water and alcohol being particularly from 1:2 to 1:20.

It also forms part of the invention a crystalline form of the amino acid salt of fosfomycin, wherein the amino acid is selected from the group consisting of lysine and histidine as defined herein, which is obtainable by any one of the processes according to the invention.

It also forms part of the invention the histidine salt of fosfomycin, which is obtainable by any one of the processes as defined herein.

The present invention also provides methods of crystallization to obtain the crystalline form of the amino acid salts of fosfomycin according to the invention. In an embodiment, optionally in combination with any of the embodiments above or below, the salt of fosfomycin according to the invention crystallizes in an aqueous alcoholic media as defined herein.

In a particular embodiment, optionally in combination with any of the embodiments above or below, the lysine salt of fosfomycin, prepared by any of the processes defined above, is further recrystallized in a mixture of water and ethanol in a volume ratio from 1:2 to 1:10, particularly about 1:5, as shown in Example 4.

In a particular embodiment, optionally in combination with any of the embodiments above or below, the histidine salt of fosfomycin, prepared by any of the processes defined above, is further recrystallized in a mixture of water and ethanol in a volume ratio from 1:2 to 1:15, particularly about 1:10, as shown in Example 3.

In another embodiment, optionally in combination with any of the embodiments above or below, a C₁-C₆ ketone, such as acetone, is also added during the crystallization process as defined herein.

The inventors have further found that a crystalline form of the arginine salt of fosfomycin, particularly in their L form as shown in the general formula (V), may be advantageous when used orally in comparison to other fosfomycin salts such as tromethamine salt of fosfomycin or the calcic salt of fosfomycin.

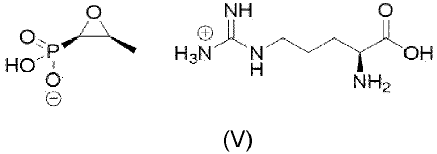

Thus, it also forms part of the invention a pharmaceutical or veterinary composition which comprises a therapeutically effective amount of a crystalline form of the arginine salt of fosfomycin, together with one or more pharmaceutically or veterinary acceptable excipients.

In a particular embodiment, optionally in combination with any of the embodiments above or below, the pharmaceutical or veterinary composition comprising a crystalline form of the arginine salt of fosfomycin is an oral composition.

In an embodiment, optionally in combination with any of the embodiments above or below, the pharmaceutical or veterinary composition which comprises a crystalline form of the arginine salt of fosfomycin as defined herein, or a crystalline form of the arginine salt of fosfomycin, is for use as antibiotic in the treatment of bacterial infections.

It is also part of the invention a crystalline form of the arginine salt of fosfomycin.

In a particular embodiment, optionally in combination with any of the embodiments above or below, the crystalline form of arginine salt of fosfomycin is L-arginine.

In another particular embodiment, optionally in combination with any of the embodiments above or below, the crystalline form comprises from 0.9 to 1.1 equivalents of arginine relative to one equivalent of fosfomycin, more particularly, the ratio of amino acid relative to fosfomycin is about 1:1.

In a particular embodiment, optionally in combination with any of the embodiments above or below, the crystalline form of the arginine salt of fosfomycin is characterized by an X-ray diffractogram that comprises characteristic peaks at 9.2, 15.4, 16.7, 17.3, 22.4, 23.8, 24.4 and 29.2 ± 0.1 degrees 2 theta measured in an X-ray diffractometer with Cu Kα radiation (1.5406 Å).

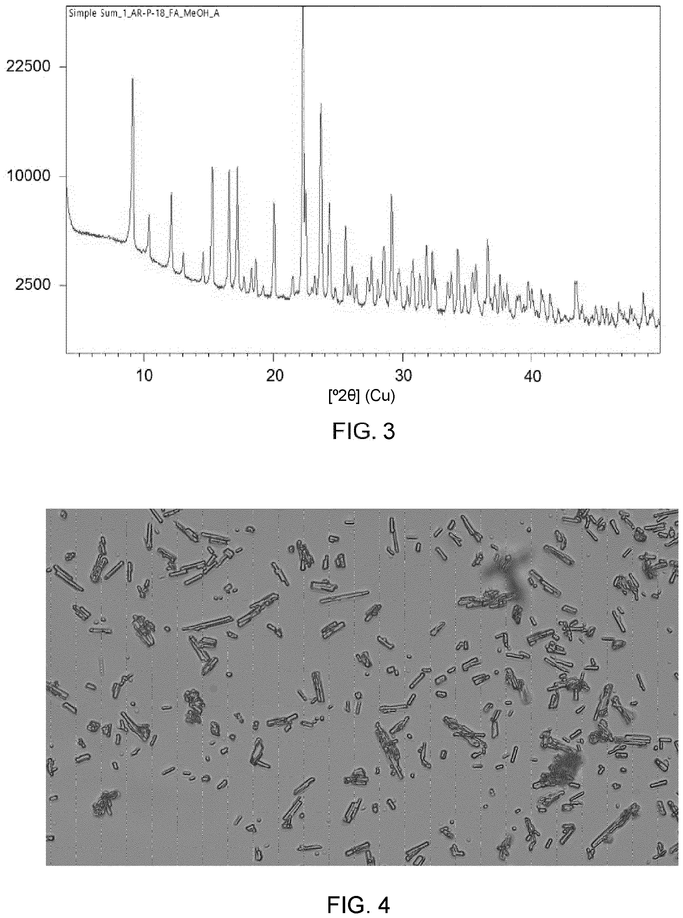

Furthermore, the arginine salt of fosfomycin is characterized by an X-ray diffractogram as shown in FIG. 3.

Moreover, the crystalline form of the arginine salt of fosfomycin is characterized by an X-ray diffractogram that comprises the characteristic peaks at the values shown in Table 3 ± 0.1 degrees 2 theta measured in an X-ray diffractometer with Cu Kα radiation (1.5406 Å).

**Table 3**

| Angle (2θ) | D Value (Å) | Rel. Intensity (%) | | Angle (2θ) | D Value (Å) | Rel. Intensity (%) |
|---|---|---|---|---|---|---|
| 9.1929 | 9.61228 | 50.61 | | 22.6143 | 3.92872 | 15.92 |
| 12.1798 | 7.26091 | 12.83 | | 23.7670 | 3.74072 | 52.87 |
| 15.3602 | 5.76391 | 21.26 | | 24.4223 | 3.64182 | 19.41 |
| 16.6639 | 5.31577 | 25.10 | | 25.6588 | 3.46905 | 14.35 |
| 17.2993 | 5.12195 | 24.82 | | 29.2355 | 3.05228 | 24.18 |
| 20.1316 | 4.40728 | 17.16 | | | | |
| 22.3812 | 3.96910 | 100.00 | | | | |

The arginine salt of fosfomycin can be prepared by analogous methods as the ones disclosed herein for the lysine salt.

It is also part of the invention a crystalline form of arginine salt of fosfomycin, as defined herein, which is obtainable by any one of the processes according to the invention.

Moreover, it is also part of the invention arginine salt of fosfomycin prepared by any of the processes defined above for the arginine salt, which is further crystallized in a mixture of water and ethanol in a volume ratio from 1:2 to 1:5, particularly about 1:2, as shown in Example 2. In a particular embodiment the water and ethanol volume ratio is about 1:2, about 1:3, about 1:4, or about 1:5.

Throughout the description and claims the word "comprise" and variations of the word are not intended to exclude other technical features, additives, components or steps. Furthermore, the word "comprise" encompasses the case of "consisting of". Additional objects, advantages and features of the invention will become apparent to those skilled in the art upon examination of the description or may be learned by practicing the invention. The following examples are provided by way of illustration, and they are not intended to be limiting of the present invention. Furthermore, the present invention covers all possible combinations of particular embodiments described herein.

### Examples

X-ray diffractometer Malvern Panalytical Empyrean
RX tube: Cu K alpha. Tube voltage: 45kV. Intensity: 40mA. Spinner= 16
Diffractogram is recorded at Measurement Range (2θ): 4-50° with Step (2θ): 0.013° and an accumulation at each point of 60s.

### Scanning Electron Microscope

Model Jeol JSM-5310.
Oxford EDS (Energy Dispersive Spectroscopy) microanalyzer with Inca Energy software.

### Example 1: L-lysine salt of fosfomycin

L-lysine hydrochloride (12 g, 66 mmol) and fosfomycin PEA salt (20 g, 79 mmol) in 32 mL of H₂O were warmed at 60-70°C. Then, 320 mL of EtOH were slowly added to make the product precipitate. The suspension was then cooled, filtered, and washed with cold EtOH. The product was recrystallized by dissolving it in 40 mL of H₂O and by adding 200 mL of EtOH. Crystalline L-lysine salt of fosfomycin was obtained in 80% yield (FIG. 1, FIG. 4). The salt obtained is in a ratio of 1:1.

### Example 2: L-arginine salt of fosfomycin

30 mL of EtOH were added to a solution of fosfomycin PEA salt (10 g, 39 mmol) in 16 mL of H₂O. The mixture was then added to a solution of L-arginine hydrochloride (6.9 g, 33 mmol) in H₂O (10 mL) at 60-70 °C. As a result, a solid precipitated and 100 mL of EtOH were then slowly added. The suspension was cooled, filtered, and washed with cold EtOH. L-arginine salt of fosfomycin was obtained in 97% yield. The product was recrystallized from a mixture of H₂O/EtOH (1:2) (FIG. 3). The salt obtained is in a ratio of 1:1.

### Example 3: L-histidine salt of fosfomycin

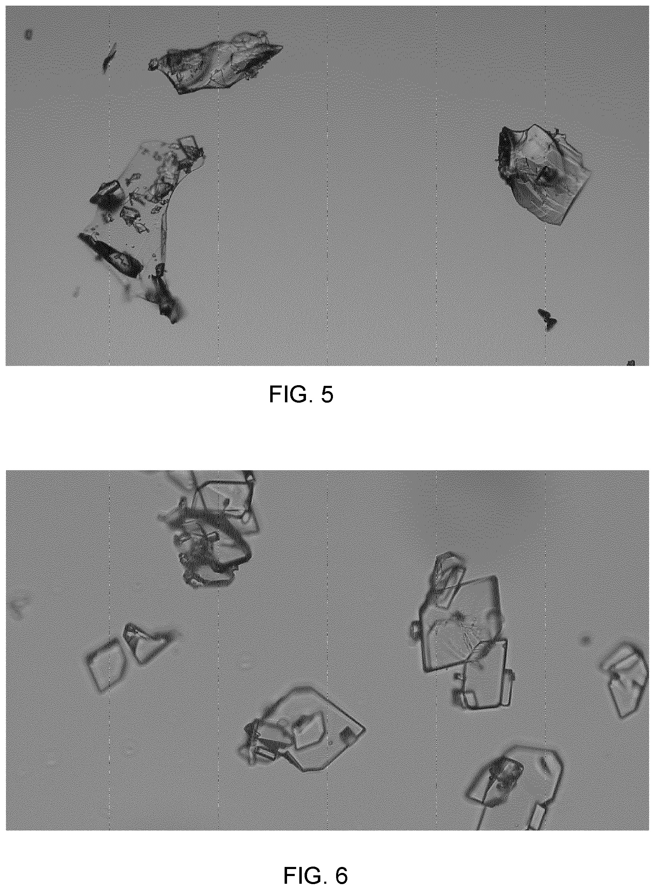

p-Toluensulphonic acid (11 g) was added over a solution of L-histidine (10 g, 64 mmol) in 25 mL of H₂O at 50 °C. This solution was slowly added over a solution of fosfomycin PEA salt (18 g, 71 mmol) in EtOH (250 mL) at 60-70 °C. The suspension was cooled, filtered, and washed with cold EtOH. The L-histidine salt of fosfomycin was recrystallized by dissolving in H₂O and adding EtOH (in a ratio of 1:10 of H₂O:EtOH). The salt obtained is in a ratio of 1:1. (FIG. 2, FIG. 5).

### Example 4: L-lysine salt of fosfomycin

NaOH (3 g) was added to a solution of fosfomycin PEA salt (10 g) in H₂O (14 mL) at 10 °C. pH was adjusted to 11-12.5 and PEA was washed out with toluene. Aqueous phase was adjusted to pH 4-4.5 with HCl and L-lysine hydrochloride (5.1 g) was added. The resulting solution was warmed to 35-40 °C and 70 mL of EtOH were added slowly. Then, the suspension was cooled and filtered to obtain L-lysine salt of fosfomycin, which was recrystallized by dissolving the solid in H₂O and adding EtOH (in a ratio of 1:5 of H₂O:EtOH) (FIG. 1, FIG. 4).

### Example 5: Synthesis of L-histidine salt of fosfomycin using an ion exchange resin

Fosfomycin PEA salt (2 g) was dissolved in 20 mL of EtOH at 0 °C, an ion exchange resin such as Amberlite IRC-120H (24 g) was added and the mixture was stirred for 30 minutes. Then, the resin was filtered out and washed with EtOH and an alcoholic solution was obtained. A saturated aqueous solution of L-histidine (1 equivalent) was slowly added onto the alcoholic solution. Next, acetone (5-100 mL) was added. The product was filtered and washed with acetone (yield: 75-88%).

### Citation List

### Patent Literature

- ES359542
- ES477167
- ES483027

## Claims

1. A pharmaceutical or veterinary composition which comprises a therapeutically effective amount of a crystalline form of an amino acid salt of fosfomycin, together with one or more pharmaceutically or veterinary acceptable excipients, wherein the amino acid is selected from the group consisting of lysine and histidine.

2. The pharmaceutical or veterinary composition according to claim 1, wherein the crystalline form of an amino acid salt of fosfomycin comprises from 0.9 to 1.1 equivalents of amino acid relative to one equivalent of fosfomycin.

3. The pharmaceutical or veterinary composition according to any of the claims 1-2, which is an injectable preparation comprising a solution of the crystalline form of an amino acid salt of fosfomycin.

4. A crystalline form of an amino acid salt of fosfomycin, wherein the amino acid is selected from the group consisting of lysine and histidine.

5. The crystalline form according to claim 4, wherein the amino acid is selected from the group consisting of L-lysine and L-histidine.

6. The crystalline form according to any of the claims 4-5, which comprises from 0.9 to 1.1 equivalents of amino acid relative to one equivalent of fosfomycin.

7. The crystalline form according to any of the claims 4-6, wherein the amino acid is lysine and the crystalline form is **characterized by** an X-ray diffractogram that comprises characteristic peaks at 9.3, 19.6, 21.2, 23.7, and 24.1 ± 0.1 degrees 2 theta measured in an X-ray diffractometer with Cu Kα radiation (1.5406 Å).

8. The crystalline form according to any of the claims 4-6, wherein the amino acid is histidine and the crystalline form is **characterized by** an X-ray diffractogram that comprises characteristic peaks at 10.5, 15.8, 21.0, 21.7 and 26.4 ± 0.1degrees 2 theta measured in an X-ray diffractometer with Cu Kα radiation (1.5406 Å).

9. A histidine salt of fosfomycin.

10. The pharmaceutical or veterinary composition as defined in any of the claims 1-3, the crystalline form as defined in any of the claims 4-8, or the histidine salt of fosfomycin as defined in claim 9, for parenteral use in therapy.

11. The pharmaceutical or veterinary composition as defined in any of the claims 1-3 the crystalline form as defined in any of the claims 4-8, or the histidine salt of fosfomycin as defined in claim 9, for use as antibiotic.

12. A process for the preparation of the crystalline form of an amino acid salt of fosfomycin as defined in any of the claims 4-7, wherein the amino acid is lysine, which comprises:
i) providing a lysine salt of fosfomycin, and
ii) recrystallizing it in a mixture of water and ethanol in a volume ratio from 1:2 to 1:10, particularly about 1:5.

13. The process according to claim 12, wherein the amino acid salt of fosfomycin of step (i) is prepared either by
a process which comprises:
a) providing a fosfomycin 1-amino-1-phenylethyl (PEA) salt,
b) treating the fosfomycin PEA salt with a base in aqueous media,
c) washing out the resulting aqueous solution with an organic solvent, and
d) adding the corresponding amino acid in protonated form to the aqueous phase;
or, alternatively,
a process which comprises:
a) providing a fosfomycin 1-amino-1-phenylethyl (PEA) salt, and
b) contacting the PEA salt with the corresponding amino acid as a free base in the presence of an acid in an aqueous alcoholic media;
or, alternatively,
a process which comprises:
a) providing a fosfomycin 1-amino-1-phenylethyl (PEA) salt, and
b) contacting the PEA salt with the corresponding amino acid in protonated form in an aqueous alcoholic media;
or, alternatively,
a process which comprises:
a) providing a fosfomycin 1-amino-1-phenylethyl (PEA) salt, and
b) contacting the PEA salt with an ionic exchange resin, and
c) reacting the resulting solution of step b) with the corresponding amino acid in an aqueous alcoholic media.

14. A process for the preparation of the crystalline form of an amino acid salt of fosfomycin as defined in any of the claims 4-7, wherein the amino acid is histidine, which comprises:
i) providing a histidine salt of fosfomycin, and
ii) recrystallizing it in a mixture of water and ethanol in a volume ratio from 1:2 to 1:15, particularly about 1:10.

15. A process for the preparation of the histidine salt of fosfomycin, which comprises:
a) providing a fosfomycin 1-amino-1-phenylethyl (PEA) salt,
b) treating the fosfomycin PEA salt with a base in aqueous media,
c) washing out the resulting aqueous solution with an organic solvent, and
d) adding histidine in protonated form to the aqueous phase;
or, alternatively,
a) providing a fosfomycin 1-amino-1-phenylethyl (PEA) salt, and
b) contacting the PEA salt with histidine as a free base in the presence of an acid in an aqueous alcoholic media;
or, alternatively,
a) providing a fosfomycin 1-amino-1-phenylethyl (PEA) salt, and
b) contacting the PEA salt with histidine in protonated form in an aqueous alcoholic media; or, alternatively,
a) providing a fosfomycin 1-amino-1-phenylethyl (PEA) salt, and
b) contacting the PEA salt with an ionic exchange resin, and
c) reacting the resulting solution of step b) with histidine in an aqueous alcoholic media.
